# EUROPEAN PATENT APPLICATION

(11) **EP 4 230 730 A1**
(43) Date of publication of application: **23.08.2023**
(21) Application number: 21878847.9
(22) Date of filing: 05.01.2021
(51) Int. Cl.: C12N 5/079

(54) **CHEMICAL CULTURE SYSTEM AND USE THEREOF**

(30) Priority: 16.10.2020 CN 202011109968; 29.12.2020 CN 202011600399
(71) Applicant: Iregene Therapeutics Ltd, Wuhan, Hubei 430000 (CN)
(72) Inventor: WEI, Jun, Wuhan, Hubei 430000 (CN); CAI, Meng, Wuhan, Hubei 430000 (CN)
(74) Representative: Goddar, Heinz J.
(86) International application number: PCT/CN2021/070250
(87) International publication number: WO 2022/077784

(57) **Abstract**

A chemical culture system and use thereof. The chemical culture system comprises a basic culture medium and a small molecule compound, and forms a serum-free culture system having a clear chemical composition. The culture medium does not use substitute serum B27 and GS21 which contain animal-derived components and are widely used currently, but uses a pure chemical molecule activation signal pathway, and uses a non-animal-derived growth factor to replace an animal-derived growth factor, and thus the composition is clear, the potential risks caused by the existence of serum and animal-derived components are avoided, and the clinical prospect of nerve cell transplantation is expanded.

## Description

### Background of the Invention

### Field of the Invention

The invention belongs to the field of cell biology, and in particular relates to a chemical culture system and use thereof.

### Description of Related Art

Neurodegenerative diseases are currently common diseases of aging, and the cost of treatment and care is extremely expensive, and there are no specific drugs on the market for effective treatment. Neurodegenerative diseases include amyotrophic lateral sclerosis (ALS), Parkinson's disease (PD), Alzheimer's disease (AD) and other diseases. According to the statistics of the World Health Organization, there will be more than 30 million patients with neurodegenerative diseases in our country in 2050, and the expected medical expenses will exceed 1 trillion. At present, the main treatment methods are to supplement or stimulate the insufficient levodopa in the brain with drugs, nerve nucleus damage surgery or deep brain stimulation surgery, etc., but none of them can achieve good curative effect, and will cause "dyskinesia" or "drug effect fluctuation" and other adverse reactions that seriously affect the quality of life.

In addition to neurodegenerative diseases, spinal cord injury (SCI) is a common traumatic disease of the nervous system. According to foreign statistics, the average cost of treatment and rehabilitation for SCI patients in their lifetime is more than $750,000, and the United States spent more than $6 billion on SCI patients every year. The prevalence rate in our country is even higher than that of developed countries in Europe and America. The high cost of treatment, long-term rehabilitation and loss of labor force have a huge impact on individuals and families, and also bring a heavy burden to society.

The difficulty in the treatment of neurodegenerative diseases and spinal cord injuries lies in the non-renewable nature of nerve cells in the central nervous system, and these diseases are caused by irreversible damage to the central nervous system. At present, the transplantation of nerve cells is the most effective treatment. Nerve cells include neural stem cells, mature neurons, astrocytes and oligodendrocytes among different nerve cell types. At present, allogeneic transplantation of neural stem cells has achieved certain clinical results. Some clinical trials adopt the method of nervous system cell line transplantation, but the existing nerve cell line resources are limited, most of which are nervous system tumor cell lines, and there are unstable factors brought about by EB (Epstein-Barr) virus during the line establishment process; whereas allogeneic neuronal cells are not easy to pass on in vitro and due to ethical restrictions and inconvenient acquisition techniques, all these factors have further hindered the clinical advancement of the above diseases. In recent years, great progress has been made in the development of embryonic stem cells and induced pluripotent stem cells (iPSC), and various types of adult cells can be obtained by using such cells for directed differentiation (Cell, 2006, 124(4) pp.663 -676; Cell, 2007, 131(5) pp.861-872). Therefore, using totipotent stem cells or pluripotent stem cells including embryonic stem cells and iPSCs as raw materials to induce differentiation of nerve cells can be used as a new idea for clinical treatment, which greatly expands the application potential of nerve cells in clinical medicine.

From pluripotent stem cells to neural stem cells, there are currently two most widely used induction methods. The first is a two-step method: first, the cells are aggregated into clusters to form embryonic bodies, and then the cells are further induced to become neuron cells. The disadvantage of this method is that the cell differentiation is not synchronized and the purity of neuron cells is not high; in addition, the use of animal-derived growth factors also restricts the clinical use of neuron cells. The second is Dual SMAD inhibition (Nat Biotechnol, 2009, 05; 27(5): 485), in which the neural stem cells obtained can differentiate into other types of neuron cells, and the principle is to mimic the signaling pathways of early embryonic development by inhibiting the BMP and TGFB pathways, thereby inducing the generation of neural stem cells. By this method, induced sensory neurons can be obtained in the presence of serum-like components (Knockout Serum Replacement) (Nat Biotechnol, 2009, 05; 27(5): 485). Although the types of cells that can be applied to transplantation have greatly increased, most of the in vitro culture of nerve cells currently relies on the presence of serum components, such as fetal bovine serum and the currently widely used substitute serum B27 series (Thermo Fisher), GS21 (G0800, Sigma-Aldrich), this type of serum contains animal-derived components, so there are a series of safety hazards, and their existence greatly limits the clinical application of nerve cell transplantation. Therefore, the development of nervous system basal medium without serum components is crucial for the development of nervous system regenerative medicine.

Based on the technical problems existing in the prior art, it is necessary to study a novel serum-free culture medium for the nervous system, instead of the currently widely used substitute serum B27 and GS21 containing animal-derived components. By using pure chemical molecules to activate signaling pathways and replacing animal-derived growth factors with non-animal-derived growth factors, the composition is clear, the clinical prospects of nerve cell transplantation are expanded.

### Summary of the Invention

In view of the above, the object of the present invention is to provide a chemical culture system, which uses pure chemical molecules to activate signal pathways, uses non-animal-derived growth factors to replace animal-derived growth factors, and avoids the potential dangers associated with the presence of serum and animal-derived components.

In order to solve the above problems, the present invention provides a chemical culture system comprising a basic culture medium and additives, the additives comprising a small molecule compound, the small molecule compound being: one or more of piperidinol oxide, lignan, D(+)-galactose, recombinant human transferrin.

Further, the small molecular compound is at least two of piperidinol oxide, luteolin, D(+)-galactose, recombinant human transferrin.

Further, the small molecular compound is piperidinol oxide, luteolin, D(+)-galactose, recombinant human transferrin.

Further, the dosage of the piperidinol oxide is 10nM-200µM; preferably 50nM, 100nM, 500nM, 1µM, 5µM, 10µM, 20µM, 50µM, 100µM, 150µM or 200µM.

Further, the dosage of the luteolin is 5µM-150µM; preferably 5µM, 10µM, 50µM, 75µM, 100µM or 150µM.

Further, the dosage of the D(+)-galactose is 5-25µg/mL; preferably 5.5µg/mL, 7.5µg/mL, 10µg/mL, 12.5µg/mL, 15µg/mL, 17.5µg/mL, 20µg/mL, 22.5µg/mL or 25µg/mL.

Further, the dosage of the recombinant human transferrin is 50-200ng/mL; preferably 55ng/mL, 75ng/mL, 100ng/mL, 125ng/mL, 150ng/mL, 175ng/mL or 200ng/mL.

Further, the basic culture medium is a DMEM medium or a DMEM/F12 medium, and the basic culture medium comprises minimum essential medium non-essential amino acids.

Further, the additives in the chemical culture system provided by the present invention further comprise growth factors and inorganic salts.

Further, the growth factors comprise vitamins, progesterone, putrescine and insulin; the inorganic salts comprise sodium chloride and sodium selenite. Further, the vitamins comprise 1µg/mL vitamin E, 1.2µM vitamin B12, 64mg/L vitamin C; the progesterone is 6.3ng/mL, the putrescine is 23µg/mL, and the insulin is 22µg/mL; the sodium chloride is 0.5g/L, and the sodium selenite is 13.6µg/L.

Another object of the present invention is to provide the use of the chemical culture system in primary nerve cell culture and nerve cell line culture.

Another object of the present invention is to provide the use of the chemical culture system in the induction of pluripotent stem cells to neural stem cells.

Another object of the present invention is to provide the use of the chemical culture system in inducing neural directional differentiation of stem cells and differentiated cell culture. Further, the use comprises astrocyte differentiation, DRG neuron differentiation culture or cortical neuron differentiation culture; Further, cytokines are added to the DRG neuron differentiation and culture process; chemical small molecule inhibitors are added during the cortical neuron differentiation culture process.

Another object of the present invention is to provide the use of the chemical culture system in the induction of brain organoids.

Compared with the prior art, the beneficial effects of the present invention are:
1. The present invention uses one or more new chemical small molecules, combined with the basic culture medium and the combination of inorganic salts and growth factors, which can support the in vitro induction of various neurons, thereby establishing a serum-free and animal-derived substance-free sensory neuron cell induction culture system, and can show better induction culture effect.
2. The neural stem cells obtained through the present medium have various complete biological functions, are stable in multiple batches, and have high purity, which solves the problems of low purity and long cycle in the production process of cell-based drugs.
3. The present invention also solves the long-standing problems of animal-derived culture methods and trophoblast contamination, and can be used for in vitro screening of drugs for nervous system diseases, so it has huge economic and social effects.

### Brief Description of the Figures

In order to more clearly illustrate the technical solutions of the examples of the present invention, the accompanying drawings used in the examples will be briefly introduced below. It should be understood that the following drawings only show some examples of the present invention, and therefore should not be regarded as a limitation on the scope, and that those skilled in the art can also obtain other related drawings based on these drawings without creative work.
Fig. 1 shows the Cyquant curves of primary cells and the human neural stem cell line ReNcell VM cultured in NouvNeu.
Fig. 2 is a schematic diagram of the results of core component screening results in NouvNeu using the human neural cell line ReNcell VM.
Fig. 3 is a graph showing the neural rosette structure and marker transcription analysis obtained after directional induction of neural stem cells using the NouvNeu system.
Fig. 4 is a schematic diagram of the expression of sensory neuron-specific molecular markers identified by immunofluorescence after neuron and glial cell induction using the NouvNeu system.
Fig. 5 is a schematic diagram of the electrophysiological activity of DRG nerve cells using the NouvNeu system.
Fig. 6 is a schematic diagram of cell activity during the process of the induction of directed differentiation of cortical neuron cells using the NouvNeu system.
Fig. 7 is a schematic diagram of the induction of brain organoids using the NouvNeu system, and the analysis of brain organoid markers and electrophysiological activity.

### Detailed Description of the Invention

The following examples are used to illustrate the invention, but do not limit the scope of the invention. With not departing from the spirit and essence of the present invention, any modifications or replacements made to the methods, steps or conditions of the present invention belong to the scope of the present invention.

In specific embodiments of the present invention, the serum-free medium means that it does not contain serum isolated directly from blood. Serum is the clear liquid portion of plasma that does not contain fibrinogen or blood cells and remains liquid after blood has clotted. The serum-free medium may contain serum substitutes. Examples of serum substitutes include purified substances such as serum albumin, transferrin, and fatty acids, which are known in the art to be substitutes for serum.

In specific embodiments of the present invention, a culture medium for induction of various cells is provided comprising a basic culture medium and additives; the additives comprising a small molecular compound, further comprising common inorganic salts and growth factors.

In specific embodiments of the present invention, the small molecular compound is one or more of piperidinol oxide, luteolin, D(+)-galactose, and recombinant human transferrin; preferably two, more preferably four.

In specific embodiments of the present invention, the basic culture medium is DMEM medium with minimum essential medium non-essential amino acids, and other mediums comprising minimum essential medium non-essential amino acids may also be selected.

In specific embodiments of the present invention, the growth factors include vitamins, progesterone, putrescine and insulin; other commonly used growth factors can also be selected for replacement or addition.

In specific embodiments of the present invention, the inorganic salts include sodium chloride and sodium selenite; other commonly used inorganic salts can also be selected for replacement or addition.

### Example 1. culture medium formulation

The formulation of NouvNeu culture medium, hereinafter referred to as NouvNeu:
Duchenne's Modified Eagle medium (DMEM medium, you can also choose serum-free DMEM-F12), Minimum Essential Medium Non-Essential Amino Acids (catalog number: 11140076, Thermo Fisher), piperidine Alcohol oxide (4-Hydroxy-TEMPO, 10nM-200µM, optimal concentration 50µM), D(+)-galactose (5-25µg/mL, optimal is 12.5µg/mL ), vitamin E (1µg/mL), vitamin B12 (1.2µM), vitamin C (L-ascorbic acid, 64mg/L), Progesterone (6.3ng/mL), putrescine (23µg/mL) , Luteolin (5µM-150µM, optimal concentration 60µM), sodium chloride (0.5g/L), sodium selenite (13.6µg/L), insulin (22µg/mL), recombinant human transferrin (50-100ng/mL, optimal concentration 100ng/mL).

At the same time, the composition was configured according to the criteria of Table 1 comparing NouvNeu culture medium under the condition that other components were unchanged.

**Table 1: NouvNeu component analysis culture medium composition list**

| | TEMPO | Luteolin | D(+)-galactose | Recombinant human transferrin |
|---|---|---|---|---|
| NouvNeu-TL | 50µM | 60µM | - | - |
| NouvNeu-TG | 50µM | - | 12.5µg/mL | - |
| NouvNeu-LT | - | 60µM | - | 100ng/mL |
| NouvNeu-TeT | 50µM | - | - | 100ng/mL |
| NouvNeu-GT | - | - | 12.5µg/mL | 100ng/mL |
| NouvNeu-LG | - | 60µM | 12.5µg/mL | - |
| NouvNeu-TLG | 50µM | 60µM | 12.5µg/mL | - |
| NouvNeu-TLT | 50µM | 60µM | - | 100ng/mL |
| NouvNeu-TGT | 50µM | - | 12.5µg/mL | 100ng/mL |
| NouvNeu-LGT | - | 60µM | 12.5µg/mL | 100ng/mL |

The control culture medium I is N2B27 culture medium, hereinafter referred to as N2B27, and its formula is: 50% Duchenne's Modified Eagle F12 medium (DMEM-F12), 50% neurobasal culture medium (Neurobasal), 0.1% N2 supplement, 2% B27 supplement.

The control culture medium II was Duchenne's Modified Eagle's medium (DMEM medium) containing 10% FBS, hereinafter referred to as FBS.

### Example 2. Culture of primary neural cells and neural cell lines and screening of compound concentrations

6-well culture plates were coated with 50µg/mL polylysine (SIGMA, P6407) and incubated for more than 3 h at 37°C in a thermostat after plate laying; then further coated with 5µg/mL Laminin (I2020, SIGMAALDRICH) and incubated for more than 3 h at 37°C in a thermostat after plate laying. Mouse primary cells and the human neural cell line ReNcell VM were cultured, where mouse primary cell isolation was performed according to the method described in Nature Protocols. Volume 7, pages 1741-1754 (2012). Cell culture conditions were 37°C, 5% carbon dioxide (Panasonic, MCO-18AC), and cell adherent culture. The culture medium of the present invention, Neuralbasal+10% FBS and N2B27 culture medium were used for comparison, respectively. Cell passage was digested with 0.05% trypsin/EDTA at 37°C for 5 min, and the cell digestion was terminated with DMEM+10% FBS. Cells were washed and centrifuged re-inoculated in T25 culture plates at a rate of 2×10⁵ per vial. Cyquant assays were performed after leaving the cells to pass 2.

The adherent culture described in step 1 to step 3 in this example is preferably performed in the presence of a basement membrane. The basement membrane of the present invention can form a thin film composed of extracellular matrix molecules on the surface of the culture vessel, which can provide support similar to that of the in vivo environment for parameters such as cell morphology, growth and differentiation, and movement. In a specific embodiment, the basement membrane is a combination of one or more of Matrigel (STEMCELL Technologies), Laminin and Vitronectin.

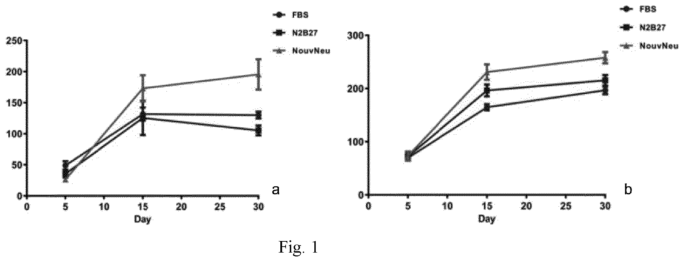

The Cyquant assay is used for quantitative detection of cell viability to compare the effects of different medium components on the physiological state of cells during long-term cell culture. The 96-well opaque cell culture plates were coated, and the cells were inoculated at 5 × 104 cells per well after the coating was completed, and three parallel replicates were set up (the average value of the three groups was used as data for calculation). The culture conditions were 37°C, 5% carbon dioxide, and fluid changes every 2½ days, using the culture medium from the known method as the control group. Samples were taken on day 1, day 5, day 15 and day 30, and cell viability assays were performed using CyQuant Kit (Invitrogen, X12223) and operated according to the instruction manual, and using SpectraMax i3 Multi-Mode Microplate Reader (VWR, model ID3-STD) for data reading. The results are shown in Fig. 1. The results show that the Cyquant experiment showed that both mouse primary cells (Figure 1a) and human neural stem cell lines (Fig. 1b) exhibited better cell viability in the NouvNeu system compared with the FBS control and the N2B27 control.

The compounds screened in the Cyquant single-product experiment were piperidinol oxide, D(+)-galactose, luteolin and recombinant human transferrin, respectively. For the concentration screening, only the concentrations of piperidinol oxide, D(+)-galactose, recombinant human transferrin and luteolin in the medium formula were changed, while the concentrations of remaining components remained unchanged.

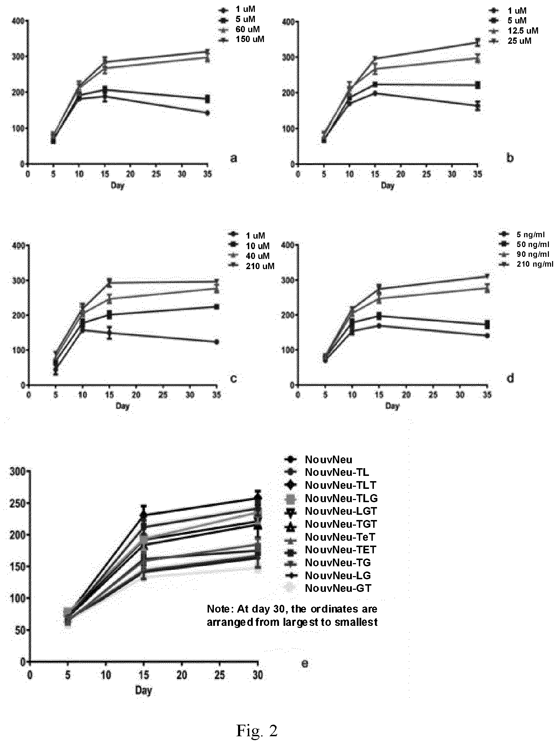

The screening results are shown in Figs. 2a-2d. Figure 2a shows that the use range of luteolin is 5µM-150µM; it is not difficult to conclude that one of 1µM, 10µM, 50µM, 75µM, 100µM and 150µM can be preferred.

Figure 2b shows that the use range of D(+)-galactose is 5-25µg/mL; it is not difficult to see that 5.5µg/mL, 7.5µg/mL, 10µg/mL, 12.5µg/mL, 15µg/mL, 17.5ug /mL, 20µg/mL, 22.5µg/mL and 25µg/mL can be used as a better dosage choice.

Figure 2c shows that the use range of piperidinol oxide is 10nM-200µM; it is not difficult to see that 50nM, 100nM, 500nM, 1µM, 5µM, 10µM, 20µM, 50µM, 100µM, 150µM or 200µM can be used as a better dosage choice.

Fig. 2d shows that the use range of recombinant human transferrin is 50-200ng/mL; it is not difficult to see that 55ng/mL, 75ng/mL, 100ng/mL, 125ng/mL, 150ng/mL, 175ng/mL or 200ng/mL can be used as a better dosage choice.

Cyquant screened single product combinations, and the culture medium was used according to the NouvNeu composition analysis medium in Table 1, and the screening results are shown in Figure 2e. The results showed that the combination of luteolin, D(+)-galactose, piperidinol oxide and human recombinant transferrin in NouvNeu showed that NouvNeu containing four components had the best cell proliferation activity among all combinations.

From the above screening results (Figure 2a-2e), the increase of the concentration of each individual component in NouvNeu can play a role in cell proliferation, so it can be judged that the composition containing one or more of luteolin, D(+)-galactose, piperidinol oxide and human recombinant transferrin can achieve cell proliferation.

**Table 2: Cyquant Counts of Primary Cell Cultures for four NouvNeu component combinations for primary cell culture (Fig. 2e)**

| | 5d | 15d | 30d |
|---|---|---|---|
| NouvNeu-TL | 70 | 212 | 231 |
| | 65 | 201 | 253 |
| | 72 | 223 | 241 |
| NouvNeu-TG | 65 | 164 | 174 |
| | 67 | 151 | 169 |
| | 59 | 170 | 182 |
| NouvNeu-TeT | 67 | 166 | 182 |
| | 69 | 143 | 195 |
| | 58 | 167 | 177 |
| NouvNeu-LG | 65 | 147 | 168 |
| | 69 | 129 | 184 |
| | 63 | 154 | 148 |
| NouvNeu-LT | 67 | 142 | 161 |
| | 69 | 153 | 176 |
| | 53 | 129 | 153 |
| NouvNeu-GT | 64 | 136 | 150 |
| | 73 | 132 | 140 |
| | 54 | 129 | 151 |
| NouvNeu-TLG | 77 | 194 | 231 |
| | 73 | 210 | 254 |
| | 81 | 176 | 221 |
| NouvNeu-TGT | 72 | 187 | 212 |
| | 71 | 198 | 239 |
| | 66 | 167 | 198 |
| NouvNeu-LGT | 70 | 192 | 231 |
| | 81 | 186 | 210 |
| | 64 | 198 | 223 |
| NouvNeu-TLT | 70 | 221 | 253 |
| | 75 | 231 | 241 |
| | 81 | 211 | 227 |
| NouvNeu | 77 | 217 | 247 |
| | 79 | 230 | 259 |
| | 63 | 246 | 268 |

### Example 3. Induced culture of neural stem cells

Human pluripotent stem cells include embryogenic pluripotent stem cells, such as H9 cell lines and human induced pluripotent stem cells. Among them, human induced pluripotent stem cells are obtained from CD34+ cells reprogrammed according to "reprogramming medium and culture method of reprogramming induced pluripotent stem cells" (Patent Publication No. CN109628383A).

Human pluripotent stem cells were coated with Matrigel (STEMCELL Technologies) in T25 cell culture flasks, and incubated in a 37°C thermostat for more than one hour after plating. According to 1×10⁶ Cells were inoculated in T25 culture flasks for expansion and passage.

For neural induction, 6-well culture plates were coated with 50µg/mL poly-Lysine (SIGMA, P6407) and incubated for more than 3 h in a 37°C thermostat after plate laying. When the pluripotent stem cells reached 70% coverage, they were digested with EDTA at 37 °C for 5 min, and the cell digestion was terminated with DMEM. After washing and centrifugation, the cells were re-inoculated into T25 culture plates in a ratio of 2×105 bottles. Nerve induction was performed using the combination of the medium and nerve induction compound of the invention, wherein the nerve induction compound was 100nM LDN-193189 HCl (Selleck, product number: S7507) and 10µM SB431542 (Selleck, product number: S1067), and the culture conditions were 37 °C, 5% CO₂ (Panasonic, MCO-18AC), and the culture medium was changed every day until the rosette structure of neural stem cells was formed.

Neural stem cells were digested with EDTA for 5 min at 37°C, and DMEM was used to terminate cell digestion. The cells were washed and centrifuged and re-inoculated in T25 culture plates coated with 50µg/mL polylysine and 5µg/mL laminin at a rate of 2 × 10⁵ per vial for expansion culture. The culture conditions are 37 °C, 5% CO₂ (Panasonic, MCO-18AC).

Neural stem cells passaged after completion of induction by the N2B27 and NouvNeu systems were taken for immunofluorescence staining identification, respectively: cells were fixed with 4% paraformaldehyde at room temperature for 40 min and washed twice with DPBS buffer; then permeabilized with 0.1% Triton X-100t for 5 min and washed twice with DPBS buffer; then the cells were incubated overnight at 4°C with DPBS buffer containing 10% horse serum and 0.1% Triton X-100; then antibodies diluted with DPBS buffer were added, incubated at 37°C for 2 h, washed with DPBS buffer for three times and photographed with Leica DMi8. The details of antibody use are shown in Table 3.

**Table 3: Antibodies used for cellular immunofluorescence staining**

| Primary antibody | Primary antibody concentration | Secondary antibody | Secondary antibody concentration |
|---|---|---|---|
| Anti-ZO1-antibody (ThermoFisher) | 1:200 | Donkey anti-mouse IgG(H+L),Alexa Fluor 568 (Invitrogen A11008) | 1:1000 |
| Anti-Sox2 antibody (CST) | 1:400 | Sheep anti-rabbit IgG(H+L),Alexa Fluor 488 (Invitrogen A11008) | 1:1000 |

Transcriptional changes of different marker genes during the induction from pluripotent stem cells to neural stem cells were detected by Q-PCR. The induced pluripotent stem cells used in Example 3, the induced neural stem cells obtained using the NouvNeu and N2B27 systems, and the cells after 3 passages using the NouvNeu component analysis medium were subjected to total RNA extraction using the RNeasy Mini or Micro Kit (QIAGEN) respectively. After extraction, 1 mg of RNA was synthesized into cDNA using SuperScript III First-Strand Synthesis System (Invitrogen). SYBR Premix Ex Taq (TaKaRa) and Thermal Cycler Dice Real Time System (TaKaRa) were used for Quan-titative PCR labeling and reaction, and beta-Actin was used as an internal reference. All data were analyzed by delta-Ct method. Each experiment was repeated three times, and variance statistics were performed. The primer sequences used to identify genes encoding different cell markers are shown in Table 4.

**Table 4: Different marker gene primers during the induction of pluripotent stem cells into neural stem cells**

| Marker | Primer Sequence |
|---|---|
| SOX2-F | CATGCAGGTTGACACCGTTGG |
| SOX2-R | ATGGATTCTCGGCAGACTGATTCA |
| PAX6-F | TCTTTGCTTGGGAAATCCG |
| PAX6-R | CTGCCCGTTCAACATCCTTAG |
| CASP3-F | GGAAGCGAATCAATGGACTCTGG |
| CASP3-R | GCATCGACATCTGTACCAGACC |
| Nestin-F | TCAAGATGTCCCTCAGCCTGGA |
| Nestin-R | TGGCACAGGTGTCTCAAGGGTAG |
| ACTIN-F | TCCCTGGAGAAGAGCTACGA |
| ACTIN-R | AGCACTGTGTTGGCGTACAG |

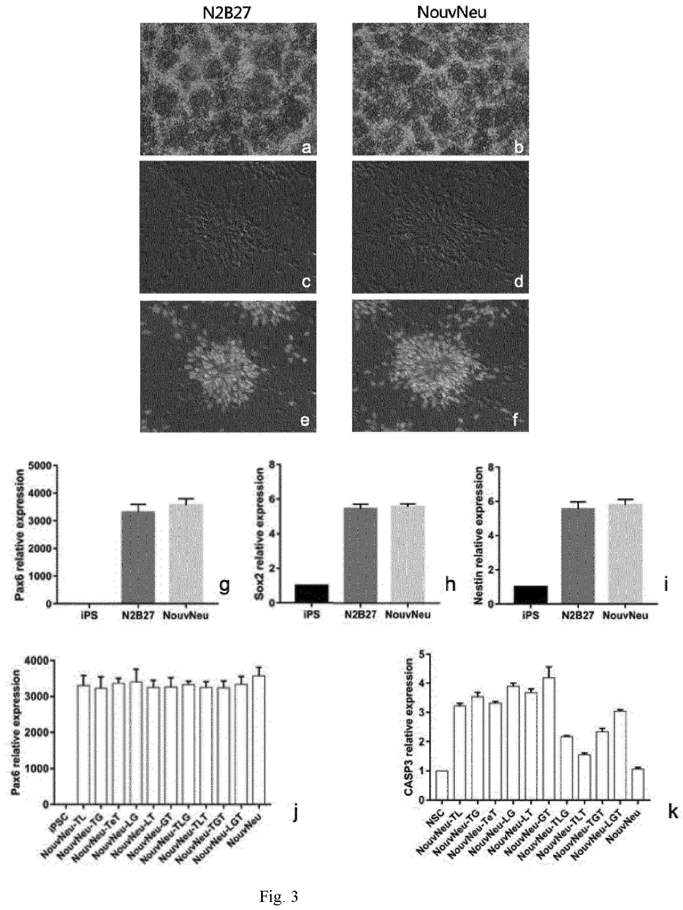

Figure 3 is a graph showing the results of neural stem cell induction using NouvNeu and the control group. Compared with the control N2B27 system (Fig. 3a, Fig. 3c), NouvNeu was able to form uniformly tiled neural rosettes (rossette) aggregates at the monolayer induction stage (Fig. 3b), and further formed a neural rosette structure after cell passage (Fig. 3d ); the neural rosette structure formed by NouvNeu (Fig. 3f) and N2B27 (Fig. 3e) controls expressed structural protein ZO1 and nuclear transcription factor SOX2 in the center of the rosette; Q-PCR analysis of RNA expression in neural stem cells, the neural stem cells induced by NouvNeu can express Pax6 (Fig. 3g), Sox2 (Fig. 3i) and Nestin (Fig. 3j). Combinations of luteolin, D(+)-galactose, piperidinol oxide and human recombinant transferrin in NouvNeu showed that all combinations could induce the expression of neural stem cell-specific marker Pax6 during neural stem cells induction. In the process of maintaining the growth of induced neural stem cells, the culture medium containing one or more combinations of luteolin, D(+)-galactose, piperidinol oxide and human recombinant transferrin can support the passage and growth of neural stem cells, but in the case of ultra-long-term culture and passage, it will cause Caspase3 expression (Fig. 3k), and the results show that NouvNeu containing four compounds can maintain neural stem cell passage and growth at lower Caspase3 expression levels.

### Example 4 Induction Differentiation and Culture of Neural Stem Cells

The neural stem cells obtained from the NouvNeu system in Example 3 were subjected to directed differentiation in NouvNeu culture medium.

### 4.1 Astrocyte differentiation

Human-induced astrocytes were differentiated using neural stem cellsobtained in Example 3 in NouvNeu basic culture medium with 10ng/mL PDGFAA (R&D Systems), 20ng/mL fibroblast growth factor 2 (Heyuan Biology), and 20ng/mL epidermal growth factor (Peprotech), Human LIF (10ng/mL, Alomone Labs), 10% fetal bovine serum (Jet Bio), and replaced with fresh culture medium every 3 days until the 21st day. Culture conditions are 37 °C, 5% CO₂ (Panasonic, MCO-18AC). The control experiment was carried out with N2B27 basic culture medium.

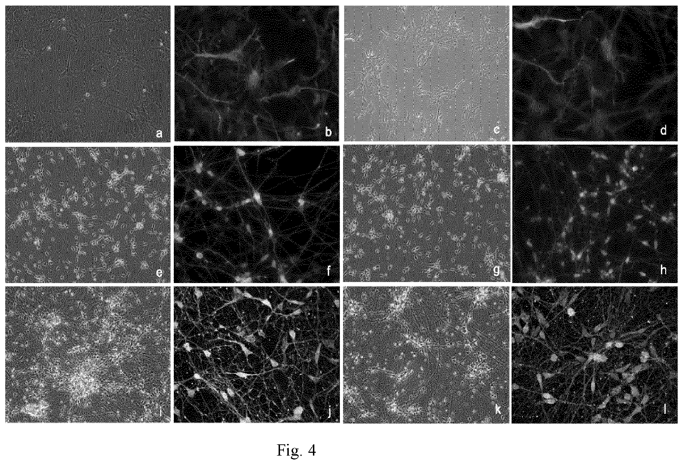

Figs. 4a-4b shows obtained using N2B27 control induction with a flattened star-like morphology and expressing the astrocyte marker GFAP; Figs. 4c-4d shows obtained using NouvNeu induction with the same cell morphology and marker expression compared with N2B27 control. The experimental results showed that both NouvNeu and N2B27 systems could differentiate into astrocytes, and the obtained astrocytes had a typical morphology and expressed the astrocyte marker MAP2 (Fig. 4a-4d).

### 4.2 Differentiation of DRG neurons

Human induced DRG neurons were differentiated using neural stem cells obtained in Example 3 by adding 3µM CHIR99021 (Selleck, catalog number: S2924), 10µM SU5402 (Tocris, catalog number: 3300/L), 10µM DAPT (Selleck, catalog number: S2215), and replaced with fresh medium every 3 days until the 21st day. Culture conditions are 37 °C, 5% CO₂ (Panasonic, MCO-18AC).

The control experiment (hereinafter referred to as CK) was carried out according to the published method (Nat Biotechnol., 2013, 30(7):715-720), and the specific operation was as follows: 820mL knockout Duchenne's modified Eagle medium (Knockout DMEM medium, catalog number: 10829018, Thermo Fisher), 150ml knockout Serum Replacement (catalog number: 10828028, Thermo Fisher), 1mM L-Glutamine (catalog number: 25030081, Thermo Fisher), 100µM Minimum Essential Medium Non-Essential Amino Acids (product number: 11140076, Thermo Fisher) and 0.1mM β-Mercaptoethanol (product number: 21985023, Thermo Fisher) were configured with the basic culture medium, and 100 nM LDN-193189 and 10 µM SB431542 were added to the basic culture medium from day 0 to day 5. Add 1% (v/v) N2 supplement (product number: 17502045, Thermo Fisher) to neurobasal medium (product number: 21103049, Thermo Fisher) to form N2 culture medium (hereinafter referred to as N2 system). N2 culture medium was added every other day in 25% increments starting from day 4 (100% N2 on day 10). On day 10, three kinds of inhibitors were added: 3µM CHIR99021 (Selleck, catalog number: S2924), 10µM SU5402 (Tocris, catalog number: 3300/1), 10µM DAPT (Selleck, catalog number: S2215), replaced with fresh medium every 3 days until day 31. The culture conditions were 37°C, 5% CO2 (Panasonic, MCO-18AC).

Figs. 4e-4f show the DRG neurons induced by N2B27, expressing the DRG neuron-specific marker sodium ion channel 1.8 and the neural cell marker Tuj; Figs. 4g-4h show the DRG neurons induced by NouvNeu, with the same cell morphology and marker expression compared with N2B27.

The experimental results showed that both NouvNeu and N2 systems could differentiate into DRG neurons, and the obtained DRG neurons had axonal structure and expressed the DRG neuron marker KCa2.2 and the neural cell marker Nestin (Figs. 4e-4h).

The induced DRG neuron cells were subjected to cell self-issuing electrical signal detection using multichannel electrodes, and the cells were cultured using NouvNeu component analysis series medium, NouvNeu culture medium, and N2B27 control culture medium, respectively, according to the DRG neuron differentiation method. A 96-well MEA system multi-channel electrode plate (AXION Biosystem, US) was coated with 100ng/mL Poly-L-lysine (Sigma-Aldrich, P4707) and placed in a cell incubator (Panasonic, MCO-18AC) with 37 °C, 5% carbon dioxide for 12 h; the poly-lysine-coated MEA multi-channel electrode plate was taken out, and the poly-lysine was sucked away. After 3 times of washing with sterilized water, PBS solution containing 3µg/mL of gelatin (Laminin, I2020, SIGMAALDRICH) was used as the nerve cell coated substrate and added to the MEA multi-channel electrode plate, and placed in a 37°C, 5% carbon dioxide cell incubator (Panasonic, MCO-18AC) for coating for 3 h. After the MEA multi-channel electrode plate was coated, the induced neuronal cells were inoculated at 5×105 cells per well. The inoculated MEA multi-channel electrode plate were placed in the MEA chamber and adjusted in the AxIS Navigator 2.0.2 software with cell culture conditions of 37°C, 5% CO₂ and run for 10 min until the chamber environment was stable. Cell spontaneous discharge signals were recorded with AxIS Navigator 2.0.2 software (AXION Biosystem, US).

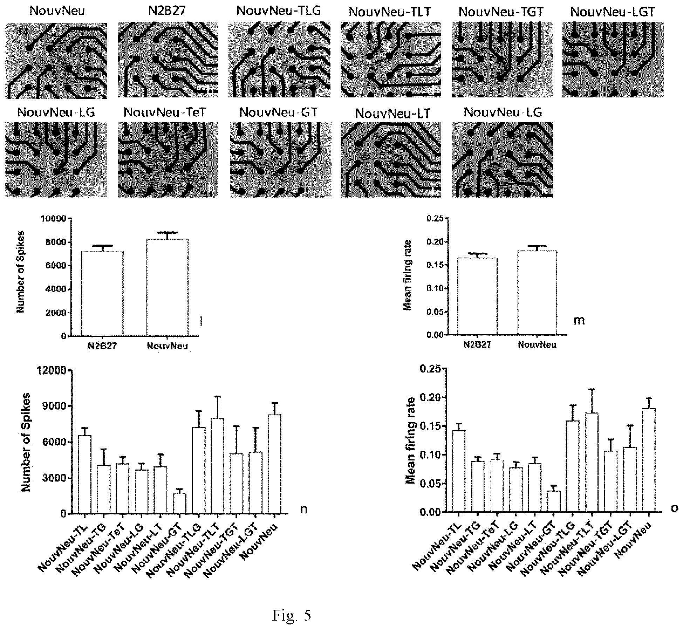

The experimental results showed that the DRG neurons induced by the NouvNeu system exhibited good electrophysiological activity. Figs. 5a-5k show the growth morphology of DRG neurons induced by the NouvNeu system and N2B27 control in the MEA electrode plate, and further use the MEA electrode plate to detect the spontaneous discharge of cells in the multi-channel electrode system, by comparing the number of cell discharges per unit time (Fig. 5i) and average firing rate (Fig. 5m), the NouvNeu system was more active than the N2B27 control. Figs. 5n-5o shows that the combination of luteolin, D(+)-galactose, piperidinol oxide and human recombinant transferrin in NouvNeu, and the results show that NouvNeu containing four components has the best cell electrophysiological activity in all combinations.

### 4.3 Cortical neuron differentiation

For cortical neuron differentiation, 6-well culture plates were coated with 50µg/mL poly-Lysine (SIGMA, P6407) and incubated for more than 3 h in a 37°C incubator after plate laying; then further coated with 3µg/mL laminin (Laminin, I2020, SIGMAALDRICH) and incubated for more than 3 h at 37°C after plate laying. The induced nerve stem cells obtained in Example 3 was re-inoculated into T25 culture plates at a rate of 1×10⁵ per vial. Neural stem cells were cultured using NouvNeu culture medium at 37°C and 5% CO₂ (Panasonic, MCO-18AC), and the medium was replaced with fresh medium every three days until neurons formation on day 30. In the control experiment, N2B27 medium was used for neuron induction, and the specific operation steps were the same as above.

Fis. 4i-4j show cortical neurons induced by N2B27, expressing the cortical neuron-specific markers MAP2 and NeuN; Figs. 4k-4l show cortical neurons induced by NouvNeu with the same cell morphology and marker expression compared with N2B27 control l.

The experimental results showed that both NouvNeu and N2B27 systems could differentiate into cortical neurons, and the obtained cortical neurons had an axonal structure and expressed cortical neuron markers NeuN and MAP2 (Figs. 4i-4l).

After induction was completed, two materials from each control group were taken separately for immunofluorescence staining identification: cells were fixed with 4% paraformaldehyde at room temperature for 40 min and washed twice with DPBS buffer; then permeabilized with 0.1% Triton X-100 for 5 min and washed twice with DPBS buffer; then the cells were incubated overnight at 4°C with DPBS buffer containing 10% horse serum and 0.1% Triton X-100; then the antibody diluted with DPBS buffer was added, incubated at 37°C for 2 h, washed three times with DPBS buffer and photographed. Details of antibody use are shown in Table 5.

**Table 5: Antibodies used for directed differentiation immunofluorescence staining**

| Primary antibody | Primary antibody concentration | Secondary antibody | Secondary antibody concentration |
|---|---|---|---|
| Anti-GFAP-antibody (SIGMA) | 1:200 | Sheep anti-mouse IgG(H+L), Alexa Fluor 488 (Abcam150113) | 1:1000 |
| Anti-NESTIN antibody (MAB5326) | 1:200 | Donkey anti-mouse IgG(H+L), Alexa Fluor 568 (Invitrogen A11008) | 1:1000 |
| Anti-KCa2.2 antibody (APC-028) | 1:50 | Sheep anti-rabbit IgG(H+L), Alexa Fluor 488 (Invitrogen A10037) | 1:1000 |
| Anti-MAP2 antibody (AB5622) | 1:1000 | Sheep anti-rabbit IgG(H+L), Alexa Fluor 405 (Abcam175673) | 1:1000 |
| Anti-NeuN antibody (MAB377) | 1:200 | Donkey anti-mouse IgG(H+L), Alexa Fluor 568 (Invitrogen A11008) | 1:1000 |

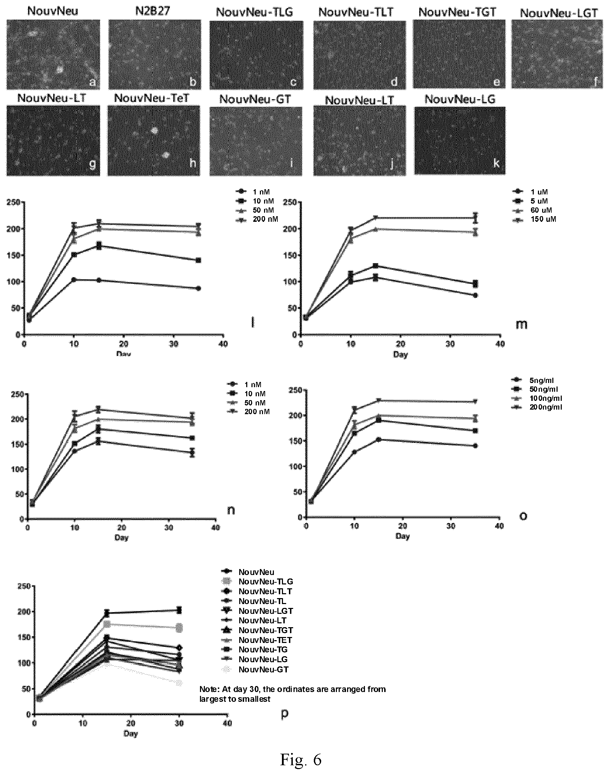

The Cyquant assay is used for quantitative detection of cell viability to compare the effects of different medium components on the physiological state of cells during long-term cell culture. The 96-well opaque cell culture plates were coated, and cells were inoculated at 5 × 10⁴ cells per well after the coating was completed, and three parallel replicates were set up (the average value of the three groups was used as data for calculation). The culture conditions were 37°C, 5% carbon dioxide, and fluid changes every 2½ days, using the culture medium from the known method as the control group. Samples were taken on day 1, day 10, day 15 and day 35, and cell viability assays were performed using CyQuant Kit (Invitrogen, X12223) and operated according to the instruction manual, and using SpectraMax i3Multi-Mode Microplate Reader (VWR, Model ID3-STD) for data reading. The results are shown in Fig. 6. Figs. 6a-6k show the growth morphology of cortical neurons induced by the NouvNeu system and N2B27 control at the same initial concentration of cells. Cyquant experiments show that NouvNeu can better maintain he cell activity during the directed differentiation of induced cortical neurons.

Figure 6l shows that the use range of piperidinol oxide is 10nM-200µM, Fig. 6m shows that the use range of luteolin is 5µM-150µM. Fig. 6n shows that the use range of D(+)-galactose is 5-25µg/mL; Fig. 6o shows that the use range of recombinant human transferrin is 50ng/mL-200ng/mL; Fig. 6p (data shown in Table 6) shows that the combination of luteolin, D(+)-galactose, piperidinol Oxide and human recombinant transferrin in NouvNeu showed that NouvNeu containing four components had the best cell proliferation activity in all combinations.

**Table 6: Cyquant counts in induction of cortical neurons by four component combinations**

| | | | |
|---|---|---|---|
| | 1d | 15d | 30d |
| NouvNeu-TL | 30 | 129 | 121 |
| | 32 | 131 | 113 |
| | 32 | 132 | 114 |
| NouvNeu-TG | 35 | 124 | 84 |
| | 27 | 121 | 92 |
| | 33 | 118 | 88 |
| NouvNeu-TeT | 27 | 116 | 92 |
| | 29 | 113 | 95 |
| | 31 | 117 | 102 |
| NouvNeu-LG | 31 | 107 | 78 |
| | 29 | 119 | 84 |
| | 33 | 104 | 87 |
| NouvNeu-LT | 32 | 153 | 101 |
| | 29 | 143 | 106 |
| | 33 | 131 | 109 |
| NouvNeu-GT | 34 | 96 | 61 |
| | 33 | 101 | 64 |
| | 30 | 99 | 59 |
| NouvNeu-TLG | 27 | 170 | 160 |
| | 37 | 180 | 174 |
| | 31 | 176 | 171 |
| NouvNeu-TGT | 32 | 117 | 98 |
| | 31 | 118 | 93 |
| | 36 | 122 | 98 |
| NouvNeu-LGT | 30 | 102 | 101 |
| | 31 | 106 | 110 |
| | 34 | 112 | 103 |
| NouvNeu-TLT | 30 | 151 | 126 |
| | 35 | 151 | 131 |
| | 31 | 143 | 131 |
| NouvNeu | 32 | 202 | 201 |
| | 34 | 198 | 209 |
| | 37 | 190 | 198 |

### Example 5 Induced culture of 3D organoids

Human induced pluripotent stem cells are used for brain organoid culture. The formula of brain organoid induction culture medium is as follows: NouvNeu basic culture medium is added with 10µM SB-431542, 100nM LDN-193189, and other inhibitors with similar effects can also be selected.

Induced pluripotent stem cells were digested into single cells using Accutase, and inoculated in U-bottom-ultra-low attachment 96-well plates according to the ratio of 9000 cells/150mL brain-like induction culture medium per well. Then, 50µM ROCK inhibitor Y27632 and 5% (v/v) heat-inactivated FBS (Life Technologies) were added, and cultured in a 5% CO₂ cell incubator (Panasonic, MCO-18AC) at 37° C. The organoid induction culture medium was replaced with fresh brain organoid induction culture medium the next day until day 10. On day 10, the brain organoids were transferred to the ultra-low-attachment 6-well plate, replaced with NouvNeu culture medium, and cultured at 80rpm on a horizontal rotator in a 5% carbon dioxide cell incubator at 37 °C until day 30.

The control experiment was carried out according to the published method (Nat Methods.2019 Nov; 16(11):1169-1175). N2B27 was used as the basic culture medium for organoid culture, and the formula of the control brain-like induction culture medium was: N2B27 basic culture medium was added with 10µM SB-431542, 100nM LDN-193189, 2µM XAV-939. The rest of the operations are the same as above.

Cellular self-issuance of electrical signals was detected in induced brain organoids using multichannel electrodes. The brain organoids induced for 30 days were digested with 10% trypsin/EDTA at 37 °C for 5-8 min, and a 96-well MEA system multi-channel electrode plate (AXION Biosystem, US) was taken, coated with 100ng/mL Poly-L-lysine (Sigma-Aldrich, P4707) in a 5% carbon dioxide cell incubator (Panasonic, MCO-18AC) at 37 °C for 12 h. The poly-lysine-coated MEA multi-channel electrode plate was removed, and the poly-lysine was absorbed. After 3 times of washing with sterilized water, PBS solution containing 3µg/mL of gelatin (Laminin, I2020, SIGMAALDRICH) was used as the nerve cell coated substrate and added to the MEA multi-channel electrode plate. The MEA multi-channel electrode plate was coated in a 37 °C, 5% CO₂ cell incubator (Panasonic, MCO-18AC) for 3 h. After the MEA multi-channel electrode plate was coated, the digested mixed cells of brain organoids were inoculated according to the number of 5×105 cells per well. The inoculated MEA multi-channel electrode plate was placed in the MEA chamber and adjusted in the AxIS Navigator 2.0.2 software with cell culture conditions of 37 °C, 5% CO₂ and run for 10 min until the chamber environment was stable. Cell spontaneous firing signals were recorded with AxIS Navigator 2.0.2 software (AXION Biosystem, US).

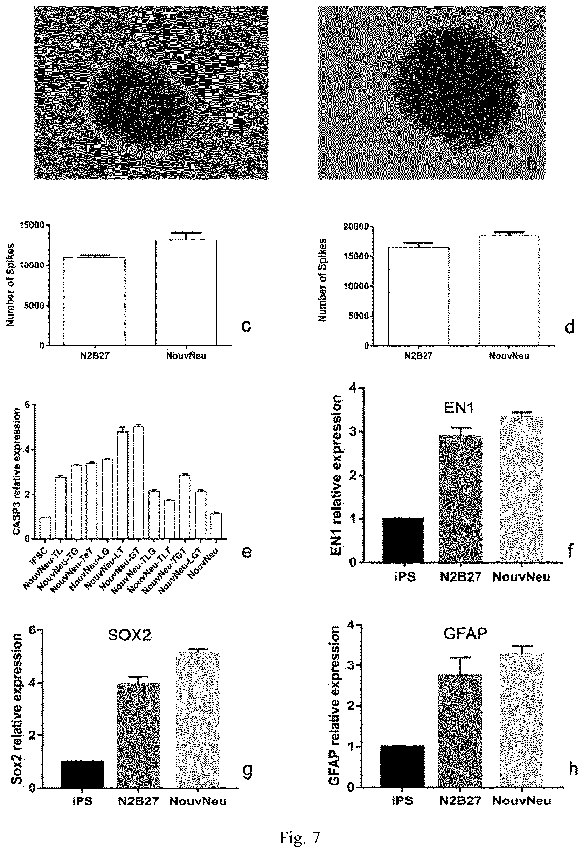

Q-PCR was used to detect the transcriptional changes of different marker genes after the formation of brain organoids: Total RNA from 18-day-old brain organoids cultured by different methods and human induced multipotent stem cells control were extracted using RNeasy Mini or Micro Kit(QIAGEN), respectively. cDNA was synthesized from 1 mg of RNA using SuperScript III First-Strand Synthesis System (Invitrogen). SYBR Premix Ex Taq (TaKaRa) and Thermal Cycler Dice Real Time System (TaKaRa) were used for Quantitative PCR labeling and reaction, and beta-Actin was used as an internal reference. All data were analyzed by delta-Ct method. Each experiment was repeated three times, and variance statistics were performed. The primer sequences used to identify genes encoding different cell markers are shown in Table 7. The results showed that the combination of luteolin, D(+)-galactose, piperidinol oxide and human recombinant transferrin in NouvNeu showed that NouvNeu containing four components had the best cell proliferation in all combinations (Fig. 7c). the brain organoids formed by the NouvNeu system and the N2B27 system can both produce glial cells, neural progenitor cells and neurons (Fig. 7d-7e).

**Table 7. Primers used for Q-PCR of markers of peripheral neurons and pluripotent stem cells:**

| | |
|---|---|
| EN1-F | GGACAATGACGTTGAAACGCAGCA |
| EN1-R | AAGGTCGTAAGCGGTTTGGCTAGA |
| SOX2-F | CATGCAGGTTGACACCGTTGG |
| SOX2-R | ATGGATTCTCGGCAGACTGATTCA |
| GFAP-F | CTGGAGAGGAAGATTGAGTCGC |
| GFAP-R | ACGTCAAGCTCCACATGGACCT |
| CASP3-F | GGAAGCGAATCAATGGACTCTGG |
| CASP3-R | GCATCGACATCTGTACCAGACC |
| ACTIN-F | TCCCTGGAGAAGAGCTACGA |
| ACTIN-R | AGCACTGTGTTGGCGTACAG |

Fig. 7 shows that: both NouvNeu (7b) and N2B27 control (7a) were able to produce brain organoids when cultured for 30 days; when spontaneous cell firing was detected using a multichannel electrical grade system, both NouvNeu systems were more active than N2B27 control by comparing the number of cell firings per unit time (Fig. 7c) and the average firing rate (Fig. 7d). The expression of RNA in brain organoid cells was analyzed by Q-PCR, and the combination of luteolin, D(+) -galactose, piperidine oxide and human recombinant transferritin in NouvNeu was performed. The results showed that NouvNeu containing four components had the best cell proliferation activity in all combinations, while some combinations resulted in massive apoptosis (Fig. 7e). Compared with the N2B27 control, the brain organoids induced by NouvNeu could express EN1 (Fig. 7f), Sox2 (Fig. 7g) and Nestin (Fig. 7h). The above results indicate that NouvNeu can maintain the growth of brain organoids and the electrophysiological activity of brain organoids.

One or more new chemical small molecules used in the above examples of the present invention, combined with the combination of basic culture medium and inorganic salts and growth factors, can support the in vitro induction of various neurons, thereby establishing a serum-free and animal-derived substance-free sensory neuron cell induction culture system, and has a better inducer culture effect than the existing animal serum-containing culture medium.

The neural stem cells obtained through the culture system of the present invention have various complete biological functions, are stable after repeated experiments, and can shorten the cycle compared with the culture medium in the prior art, and have great application prospects.

The present invention does not use substitute serum B27 and GS21 containing animal-derived components, uses pure chemical molecules to activate signal pathways, and uses non-animal-derived growth factors to replace animal-derived growth factors with clear composition. It solves the long-standing problems of animal-derived culture methods and trophoblast contamination, and can be used for in vitro screening of drugs for nervous system diseases, so it has huge economic and social effects.

The present invention is not limited to the description in the description and the implementation, so the additional advantages and improvements can easily be realized by those skilled in the art, so without departing from the spirit and scope of the general concept defined by the claims and equivalent scope, the invention is not limited to the specific details, representative schemes, and graphical examples shown and described herein.

## Claims

1. A chemical culture system **characterized by** comprising a basic culture medium and additives; the additives comprise a small molecular compound, the small molecular compound is one or more of piperidinol oxide, luteolin, D(+)-galactose, recombinant human transferrin; the dosage of the piperidinol oxide is 10nM-200µM; the dosage of the luteolin is 5µM-150µM; the dosage of the D(+)-galactose is 5-25µg/mL; the dosage of the recombinant human transferrin is 50-200ng/mL.

2. The chemical culture system according to claim 1, wherein the small molecular compound is at least two of piperidinol oxide, luteolin, D(+)-galactose, recombinant human transferrin.

3. The chemical culture system according to claim 1, wherein the small molecule compound is piperidinol oxide, luteolin, D(+)-galactose, recombinant human transferrin.

4. The chemical culture system according to claim 1, wherein the dosage of the piperidinol oxide is 50nM, 100nM, 500nM, 1µM, 5µM, 10µM, 20µM, 50µM, 100µM, 150µM or 200µM.

5. The chemical culture system according to claim 1, wherein the dosage of the luteolin is 5µM, 10µM, 50µM, 75µM, 100µM or 150µM.

6. The chemical culture system according to claim 1, wherein the dosage of the D(+)-galactose is 5.5µg/mL, 7.5µg/mL, 10µg/mL, 12.5µg/mL, 15µg/mL, 17.5µg/mL, 20µg/mL, 22.5µg/mL or 25µg /mL.

7. The chemical culture system according to claim 1, wherein the dosage of the recombinant human transferrin is 55ng/mL, 75ng/mL, 100ng/mL, 125ng/mL, 150ng/mL, 175ng/mL or 200ng/mL.

8. The chemical culture system according to claim 1, wherein the basic culture medium is DMEM medium or DMEM-F12 medium, and the basic culture medium is supplemented with minimum essential medium non-essential amino acids.

9. The chemical culture system according to claim 1, wherein the additives further comprise growth factors and inorganic salts.

10. The chemical culture system according to claim 9, wherein the growth factors comprise vitamins, progesterone, putrescine and insulin; the inorganic salts comprise sodium chloride and sodium selenite.

11. The chemical culture system according to claim 10, wherein the vitamins comprise 1µg/mL vitamin E, 1.2µM vitamin B12, 64mg/L vitamin C; the progesterone is 6.3ng/mL, the putrescine is 23µg/mL, and the insulin is 22µg/mL; the sodium chloride is 0.5g/L, and the sodium selenite is 13.6µg/L.

12. Use of the chemical culture system described in any one of claims 1-11 in primary nerve cell culture and nerve cell line culture.

13. Use of the chemical culture system described in any one of claims 1-11 in the induction of pluripotent stem cells to neural stem cells.

14. Use of the chemical culture system described in any one of claims 1-11 in inducing neural directed differentiation of stem cell and differentiated cell culture.

15. The use according to claim 14, wherein the use comprises astrocyte differentiation, DRG neuron differentiation culture or cortical neuron differentiation culture.

16. The use according to claim 15, wherein cytokines are added to the DRG neuron differentiation and culture process.

17. The use according to claim 15, wherein chemical small molecule inhibitors are added during the cortical neuron differentiation culture process.

18. Use of the chemical culture system described in any one of claims 1-11 in the induction of brain organoids.
